# EUROPEAN PATENT APPLICATION

(11) **EP 3 485 873 A1**
(43) Date of publication of application: **22.05.2019**
(21) Application number: 18206592.0
(22) Date of filing: 15.11.2018
(51) Int. Cl.: A61K 9/00, A61K 47/10, A61K 47/12, A61K 47/18, A61K 31/00

(54) **STABLE PHARMACEUTICAL INJECTABLE COMPOSITIONS OF MICAFUNGIN**

(30) Priority: 17.11.2017 IN 201721041261
(71) Applicant: Cadila Healthcare Limited, Ahmedabad, Gujarat 380015 (IN)
(72) Inventor: KULKARNI, Sushrut Krishnaji, 382210 Gujarat (IN); SINGH, Debjani Manoj, 382210 Gujarat (IN); NAHATA, Tushar Surajmal, 382210 Gujarat (IN); CHOUHAN, Pradeep Jawarchandra, 382210 Gujarat (IN)
(74) Representative: Hamer, Christopher K.

(57) **Abstract**

The present invention relates to stable pharmaceutical compositions of micafungin or a pharmaceutically acceptable salt thereof suitable for parenteral administration. In particular, the invention relates to stable injectable compositions of micafungin or a pharmaceutically acceptable salt thereof which does not require reconstitution. The invention also relates to processes for preparing such compositions and use thereof for the treatment of fungal infections.

## Description

### FIELD OF THE INVENTION:

The present invention relates to stable pharmaceutical compositions of micafungin or a pharmaceutically acceptable salt thereof suitable for parenteral administration. In particular, the invention relates to stable injectable compositions of micafungin or a pharmaceutically acceptable salt thereof which does not require reconstitution. The invention also relates to processes for preparing such compositions and use thereof for the treatment of fungal infections.

### BACKGROUND OF THE INVENTION:

Micafungin is echinocandin class anti-fungal agent first time disclosed in European patent application 0788511. Micafungin is represented by formula I shown below:

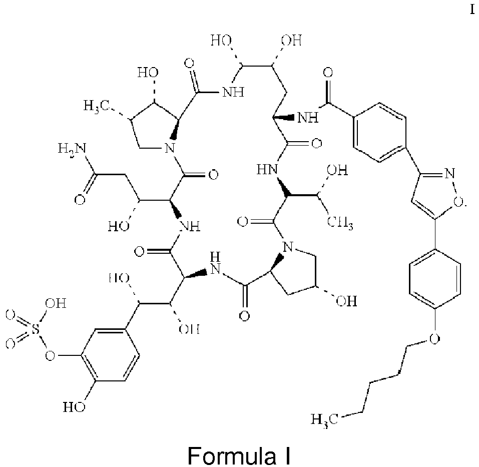

Micafungin sodium has been approved for the treatment of esophageal candidiasis, and for the prophylaxis of candida infections in patients undergoing hematopoietic stem cell transplantation. Micafungin sodium has a unique mechanism of action that inhibits the synthesis of 1, 3-*β*-D-glucans in the fungal cell wall. The drug was first launched in Japan in December 2002 as Fungard™. It was then also approved as Mycamine™ by the US Food and Drug Administration in March 2005.

Since micafungin and salts thereof are generally unstable to light, heat, humidity, acid, and the like, it is desired to develop pharmaceutical compositions in which micafungin and salts thereof are stabilized.

U.S. Patent No. 6,774,104 discloses stable lyophilized compositions of micafungin and lactose.

U.S. Patent No. 7,112,565 discloses formulating stable lyophilized compositions of micafungin with disaccharide, polysaccharide or sodium chloride.

However, compared to the liquid products, lyophilization manufacturing process is considerably more expensive, with significant capital investment and potential for long manufacturing cycle times. The development of lyophilized drug product typically requires more expensive process characterization than a liquid composition in order to develop well characterized lyophilized cycle and to obtain a sophisticated understanding of the effect of individual lyophilization cycle parameters on the quality of the drug product. Beyond simply measuring percent moisture of the lyophilized cake on stability, increased analytical testing is required to understand the effect of residual moisture on cake structure and product stability. Solid-state characterization techniques, including near infrared (NIR), X-ray diffraction, fourier transformed infrared (FTIR) and Raman spectroscopy should be employed throughout the process development to understand how the process impacts the critical quality attributes of the drug product candidate.

International (PCT) Publication No. 2012103802 discloses liquid pharmaceutical compositions of micafungin in aqueous solvent using at least one stabilizer such as monosaccharide, disaccharide or polysaccharide, or combinations thereof; preferably, lactose, sucrose, maltose, trehalose or combinations thereof. This document discloses liquid pharmaceutical compositions of micafungin containing water as the only vehicle i.e., substantially water based composition, free of any organic and/or non-aqueous vehicle.

Hence, there still remains a need for alternate stable liquid pharmaceutical compositions of micafungin suitable for parenteral administration.

### SUMMARY OF THE INVENTION:

In one general aspect, the present invention relates to a stable liquid pharmaceutical composition suitable for parenteral administration comprising micafungin or a pharmaceutically acceptable salt thereof, and one or more pharmaceutically acceptable excipients.

In another aspect there is provided a stable liquid pharmaceutical composition suitable for parenteral administration comprising micafungin or a pharmaceutically acceptable salt thereof and one or more non-aqueous solvents.

In another aspect there is provided a stable liquid pharmaceutical composition suitable for parenteral administration comprising micafungin or a pharmaceutically acceptable salt thereof in an aqueous solvent, wherein the composition does not contain any stabilizer, for example, monosaccharide, disaccharide or polysaccharide, for example, lactose, sucrose, maltose or trehalose.

In another aspect there is provided a stable liquid pharmaceutical composition suitable for parenteral administration comprising micafungin or a pharmaceutically acceptable salt thereof and one or more non-aqueous polar protic solvents.

In another aspect there is provided a stable liquid pharmaceutical composition suitable for parenteral administration comprising micafungin or a pharmaceutically acceptable salt thereof and one or more polar aprotic solvents.

In another aspect there is provided a stable liquid pharmaceutical composition suitable for parenteral administration comprising micafungin or a pharmaceutically acceptable salt thereof and a mixture of polar aprotic and non-aqueous polar protic solvents.

In another aspect there is provided a stable liquid pharmaceutical composition suitable for parenteral administration comprising micafungin or a pharmaceutically acceptable salt thereof, one or more of non-aqueous polar protic solvents and water.

In another aspect there is provided a stable liquid pharmaceutical composition suitable for parenteral administration comprising micafungin or a pharmaceutically acceptable salt thereof and a mixture of propylene glycol and water, wherein the composition does not contain any stabilizer, for example, monosaccharide, disaccharide or polysaccharide, for example, lactose, sucrose, maltose or trehalose, or the combinations thereof.

In another aspect there is provided a stable liquid pharmaceutical composition comprising micafungin or a pharmaceutically acceptable salt thereof and one or more pharmaceutical excipients, wherein the composition retains at least about 80% of the potency of micafungin or a pharmaceutically acceptable salt thereof in the pharmaceutical composition after storage for three months at 2-8°C.

The details of one or more embodiments of the invention are set forth in the description below. Other features of the invention will be apparent from the description.

### DETAILED DESCRIPTION OF THE INVENTION:

The inventors of the present invention found that the micafungin is heat sensitive and shows temperature dependant degradation in liquid state and it is possible to develop a stable non-aqueous liquid composition of micafungin suitable for parenteral administration if it is stored at 2-8°C.

The inventors of the present invention have found that storage stable liquid pharmaceutical compositions of micafungin or a salt thereof can be prepared by dissolving micafungin in non-aqueous solvent and controlling the dissolved oxygen level (less than 4 ppm) and headspace level less than 10% of oxygen.

The term "micafungin" used throughout the specification refers to micafungin *per se,* pharmaceutically acceptable salts, pharmaceutically acceptable solvates, pharmaceutically acceptable hydrates, pharmaceutically acceptable enantiomers, pharmaceutically acceptable derivatives, pharmaceutically acceptable polymorphs and pharmaceutically acceptable prodrugs thereof, its various crystalline or amorphous forms or mixtures thereof.

The composition of the present invention comprises micafungin sodium in a concentration in the range from about 1 mg/mL to about 500 mg/mL, for example, in the range from about 5 mg/mL to about 300 mg/mL, in the range from about 10 mg/mL to about 200 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL or 190 mg/mL. The stable non-aqueous pharmaceutical composition of the present invention is a liquid concentrate which may be further diluted using suitable fluid before parenteral administration.

The term "stable" refers to both the physical and chemical stability of a composition in any form, such as a solution. A composition is stable if it exhibits minimal change over the time relative to when it is manufactured. Stability is measured at various time points through a planned product expiration date with evaluation criteria including such items as therapeutic activity, appearance, levels of particulate matter, pH, content of active ingredient(s), and levels of degradation products, impurities, or related substances.

The term "physical stability" with respect to clear solution refers to maintenance of clear state without any drug precipitation, maintenance of color or colorless state and/or maintenance of dissolved oxygen level.

The term "chemical stability" relates to maintenance of drug-related impurities in terms of total impurities, known impurities, single maximum known impurity and single maximum unknown impurity, within the allowed limits by the regulatory agency.

The term "suitable for parenteral administration", as used herein with respect to the mode of administration, includes all modes of administration other than oral ingestion. Examples of "parenteral" administration include intravenous, subcutaneous or intramuscular administration where the micafungin composition is generally a solution that is formulated to enter the body of the patient either through an injection via syringe or intravenously, through an IV tube, or other invasive modes known to the artisan.

The term "inert gas" refers to a gas which expels or removes the dissolved oxygen content from the composition and maintains inert gaseous atmospheric environment in the composition. Examples of inert gas include nitrogen or carbon dioxide.

In one embodiment of the present invention, there is provided a liquid pharmaceutical composition for parenteral administration comprising micafungin or a pharmaceutically acceptable salt thereof, one or more non-aqueous solvents and optionally water. The composition may be in the form of ready to use or ready to dilute liquid suitable for parenteral administration. The composition may be in the form of clear solution, suspension, or emulsion.

In another embodiment of the present invention, there is provided a liquid pharmaceutical composition for parenteral administration comprising micafungin or a pharmaceutically acceptable salt thereof, one or more non-aqueous solvents and water. The composition may comprise non-aqueous solvent and water in a ratio between 1:9 and 9:1, for example, between 1:8 and 8:1, between 1:7 and 7:1, between 1:6 and 6:1, between 1:5 and 5:1, between 1:4 and 4:1, between 1:3 and 3:1, between 1:2 and 2:1 or about 1:1.

It is preferred that compositions of the present invention are provided in non-aqueous liquid solution. The solutions of the present invention are subjected to parenteral administration.

In one embodiment, there is provided a stable non-aqueous pharmaceutical composition suitable for parenteral administration comprising micafungin or a pharmaceutically acceptable salt thereof and one or more non-aqueous solvents.

In another embodiment, there is provided a stable liquid pharmaceutical composition suitable for parenteral administration comprising micafungin or a pharmaceutically acceptable salt thereof in an aqueous solvent without any stabilizer. The composition as per this embodiment is devoid of a stabilizer selected from monosaccharide, disaccharide or polysaccharide preferably devoid of one or more of the lactose, sucrose, maltose and trehalose.

In another embodiment, there is provided a stable non-aqueous pharmaceutical composition suitable for parenteral administration comprising micafungin or a pharmaceutically acceptable salt thereof and one or more polar aprotic solvents.

In another embodiment, there is provided a stable non-aqueous pharmaceutical composition suitable for parenteral administration comprising micafungin or a pharmaceutically acceptable salt thereof and one or more non-aqueous polar protic solvents.

In another embodiment, there is provided a stable non-aqueous pharmaceutical composition suitable for parenteral administration comprising micafungin or a pharmaceutically acceptable salt thereof and a mixture of polar aprotic solvent and one or more non-aqueous polar protic solvents.

Such compositions may typically comprise 90% or less, by volume of the composition, of the non-aqueous polar protic solvent.

In another embodiment, there is provided a stable non-aqueous pharmaceutical composition suitable for parenteral administration comprising micafungin or a pharmaceutically acceptable salt thereof, one or more non-aqueous solvents and one or more pharmaceutically acceptable excipients.

Suitable polar aprotic solvents which may be used in the present invention include, but not limited to, one or more of 1-methyl-2-pyrrolidone, 1, 3-dimethyl-2-imidazolidinone, dimethylacetamide, dimethyl sulfoxide, acetone, tetrahydrofuran, 1, 4-dioxane, acetonitrile, dimethyl formamide or propylene carbonate.

Suitable non-aqueous polar protic solvents of the present invention include, but not limited to, one or more of the glycerin; alkyl alcohols, for example, ethanol, ethylene glycol, propylene glycol, butylene glycol; polysorbates, for example TWEEN 20, TWEEN 40, and TWEEN 80; cyclodextrin derivatives, for example hydroxypropyl-*β*-cyclodextrin; polyalkylene glycols, for example, various grades of polyethylene glycol like polyethylene glycol 300, polyethylene glycol 400 and polyethylene glycol 600, polypropylene glycol, polybutylene glycol; or primary amides, for example niacinamide.

In another embodiment, the composition of the present invention comprises one or more pharmaceutically acceptable excipients selected from the group consisting of an antioxidant, a surfactant, a lipid, an organic acid, a preservative, a chelating agent, a pH adjusting agent, a complexing agent or a combination thereof.

Suitable antioxidants which may be used in the present invention include, but not limited to, monothioglycerol, ascorbic acid and its derivatives, cysteine hydrochloride monohydrate, butylated hydroxy anisole, butylated hydroxy toluene, alkyl gallate, sodium meta-bisulfite, sodium bisulfite, sodium metabisulfite, sodium dithionite, sodium thioglycollic acid, sodium formaldehyde sulfoxylate, tocopherol and derivatives thereof, and sodium sulfite. The most preferred antioxidant is monothioglycerol. The amount of an antioxidant may range from about 0.01% w/v to about 10.0% w/v of the composition.

Suitable surfactants which may be used in the present invention include, but not limited to, polysorbates, their ether ethoxylates, produced by reaction of sorbitan esters with ethylene oxide, polyoxyethylene alkyl phenol, polyoxyethylene cetyl ether, polyoxyethylene alkyl-aryl ether, polyoxyethylene monolaurate, polyoxyethylene vegetable oil, polyoxyethylene sorbitan monolaurate, polyoxyethylene esters or mixed fatty and resin acids, polyoxyethylene sorbitol lanolin derivative, polyoxyethylene tridecylether, polyoxyethylene sorbitan esters of mixed fatty and resin acids, polyoxyethylene sorbitan monostearate, polyoxyethylene sorbitan monooleate, polyoxyethylene monostearate, polyoxyethylene stearyl ether, polyoxyethylene oleyl ether, polyoxyethylene tridecyl ether, polyoxyethylene fatty alcohol, polyoxyethylene alkyl amine, polyoxyethylene glycol monopalmitate, polyoxyethylene sorbitan monopalmitate, polyoxyethylene cetyl ether, polyoxyethylene oxypropylene stearate, polyoxyethylene lauryl ether, polyoxyethylene lanolin derivative, sodium oleate, quaternary ammonium derivative, potassium oleate, N-cetyl N-ethyl morpholinium ethosulfate, sodium lauryl sulfate or mixtures thereof.

Suitable lipids which may be used in the present invention include, but not limited to, dimyristoyl phophatidylcholine (DMPC), Dimyristoyl phosphatidylglycerol (DMPG) or distearoylphophatidylglycerol (DSPG).

Suitable organic acids which may be used in the present invention include, but not limited to, citric acid, lactic acid, malic acid or benzoic acid.

Suitable preservatives which may be used in the present invention include, but not limited to, phenol, m-cresol, methyl p-hydroxybenzoate (methyl paraben), propyl p-hydroxybenzoate (propyl paraben), 2-phenoxyethanol, butyl p-hydroxybenzoate, 2-phenylethanol, benzyl alcohol, chlorobutanol, and thiomerosal, or mixtures thereof. The amount of a preservative may range from about 0.01% w/v to about 10.0% w/v of the composition.

Suitable chelating agents which may be used in the present invention include, but not limited to, citric acid monohydrate, EDTA, sodium ascorbate, pentetic acid or mixture thereof.

Suitable pH adjusting agents which may be used in the present invention include, but not limited to, sodium hydroxide, hydrochloric acid, organic acids, tris-buffer, acidic amino acids, basic amino acids, sodium carbonate, sodium bicarbonate, magnesium oxide, meglumine or mixtures thereof.

Suitable complexing agents which may be used in the present invention include, but not limited to, niacinamide, nicotinic acid, creatine or cyclodextrins.

In another embodiment, there is provided a non-aqueous liquid injectable composition of micafungin having less than 5% of micafungin degradation impurities.

In another embodiment of the invention, there is provided a liquid pharmaceutical composition for parenteral administration comprising micafungin or a pharmaceutically acceptable salt thereof which does not contain more than 1% of Impurity I, more than 1% of Impurity II, more than 1% of Impurity III, more than 1% of Impurity IV, more than 1% of Impurity V, more than 1% of Impurity VI, more than 1% of Impurity VII, more than 1% of Impurity at RRT 1.04 and more than 1% of Impurity at RRT 0.77, more than 1% of the single maximum unknown impurity and/or more than 2% (for example, 1% or 0.5%) of the total impurities when stored at 2-8°C temperature, for more than 1 month, for example, for 3 months, for 6 months, for 12 months, for 18 months, for 24 months or for 36 months as determined by high performance liquid chromatography ("HPLC").

In another embodiment of the invention, there is provided a liquid pharmaceutical composition for parenteral administration comprising micafungin or a pharmaceutically acceptable salt thereof which does not contain more than 3%, for example, does not contain more than 2%, does not contain more than 1% or does not contain more than 0.5% of the total impurities when stored at 2-8°C temperature, for more than 1 month, for example, for 3 months, for 6 months, for 12 months, for 18 months, for 24 months or for 36 months as determined by high performance liquid chromatography ("HPLC").

In one embodiment, there is provided a stable liquid pharmaceutical composition for parenteral administration comprising micafungin or a pharmaceutically acceptable salt thereof, which remains stable for commercially relevant time period after manufacturing, such as for about 1, 3, 6, 12, 18, 24 or 36 months, when it is kept in its original packaging under the specified storage conditions.

In another embodiment, there is provided a stable liquid pharmaceutical composition for parenteral administration comprising micafungin or a pharmaceutically acceptable salt thereof, which may retain at least about 90% potency, for example, at least about 95% potency or at least about 99% potency of the drug when stored at 2-8°C temperature, for more than 1 month, for example, for 3 months, for 6 months, for 12 months, for 18 months, for 24 months or for 36 months.

In another embodiment, there is provided a stable liquid pharmaceutical composition for parenteral administration comprising micafungin or a pharmaceutically acceptable salt thereof, which remains stable and compliant as per ICH guideline for impurities for respective product, for more than 2 months, for example, for 3 months, for 6 months, for 12 months, for 24 months or for 36 months, when stored at 2-8°C temperature.

In another embodiment, there is provided a stable liquid pharmaceutical composition for parenteral administration comprising micafungin or a pharmaceutically acceptable salt thereof, which does not form precipitate and remains physically stable for more than 2 months, for example, for 3 months, for 6 months, for 12 months, for 24 months or for 36 months, when stored at 2-8°C temperature.

In one embodiment, there is provided a method for preparing liquid pharmaceutical composition for parenteral administration comprising micafungin comprising deoxygenation of the liquid by bubbling with one or more inert gas until the oxygen content is below 4 ppm, for example, below 3 ppm, below 2 ppm, below 1 ppm or below 0.5 ppm.

In one embodiment, there is provided a method for preparing liquid pharmaceutical composition for parenteral administration comprising micafungin comprising deoxygenation of the liquid by placing it under vacuum, until the oxygen content is below 4 ppm, for example, below 3 ppm, below 2 ppm, below 1 ppm or below 0.5 ppm.

In another embodiment, the deoxygenation of liquid pharmaceutical composition for parenteral administration comprising micafungin having an oxygen concentration below 4 ppm, for example, below 3 ppm, below 2 ppm, below 1 ppm or below 0.5 ppm, may be performed by one or more of (a) topping micafungin with an inert gas atmosphere heavier than air, (b) placing the composition in a closed container in which the prevailing pressure is less than the atmospheric pressure, for example less than 1,00,000 Pa, 90,000 Pa, 80,000 Pa, 70,000 Pa, 60,000 Pa, 50,000 Pa or 40,000, (c) deoxygenation of the composition is completed by addition of an antioxidant, (d) protecting composition from possible resorption of oxygen during the course of production, by keeping it in an inert gas atmosphere, (e) keeping the composition into such bottles/vials/ampules which are previously cleared of air by insufflation with inert gas, and notably topping gas which is heavier than air, for example, argon.

In another embodiment, there is provided a stable non-aqueous pharmaceutical composition of micafungin suitable for parenteral administration, wherein it retains at least 80% of the potency of micafungin or a pharmaceutically acceptable salt thereof in the pharmaceutical composition after storage for three months at 2-8°C.

In another embodiment, the liquid composition of micafungin is prepared by:
i) preparing the solution of micafungin or a pharmaceutically acceptable salt thereof, one or more non-aqueous solvents and one or more pharmaceutically acceptable excipients;
ii) sparging the solution of step i) with a pharmaceutically inert gas;
iii) sterilizing the solution of step ii) and,
iv) filling the resulting solution into the suitable container with headspace of pharmaceutically inert gas.

The micafungin composition may be sterilized in order to comply with regulatory standards and also to ensure safe delivery of micafungin in therapeutic effective concentration. Sterilization renders the composition free from viable pathogens and ensures a low endotoxin level in compliance with regulatory standards. Sterilization may be accomplished by conventional methods and techniques. For example, solution compositions may be sterilized by sterile filtration, irradiation, heating, or autoclaving of the solution. Other liquid compositions, such as suspensions, emulsions, and the like, may also be sterilized by conventional methods including, without limitation, irradiation, heating, autoclaving, or a combination thereof.

In another embodiment, there is provided a method of treating fungal infection comprising:
i) identifying a patient in need of treatment of fungal infection;
ii) providing a liquid pharmaceutical composition comprising a therapeutically effective amount of micafungin or a pharmaceutically acceptable salt thereof and a non-aqueous solvent;
iii) diluting the liquid pharmaceutical composition with a pharmaceutically acceptable injectable diluent to form a pharmaceutical preparation;
iv) administering the pharmaceutical preparation to the patient in need of treatment.

More particularly, the fungal infections include candidemia, acute disseminated candidiasis, candida peritonitis, abscesses and esophageal candidiasis.

Here, pharmaceutically acceptable injectable diluent may be 0.9% of sodium chloride or 5% dextrose solution.

In one another embodiment, the present invention provides process for preparing a stable liquid pharmaceutical composition comprising steps of:
a. providing non-aqueous solvent in a glass beaker,
b. optionally adding preservative to "step a" solvent to get a clear solution,
c. optionally adding an anti-oxidant to "step a" solvent or to "step b" solution,
d. adding micafungin sodium to "step a" solvent or to "step b" or "step c" solution to get a clear solution,
e. optionally adjusting pH of "step d" solution in the range of pH 5.0-7.0 using suitable pH adjusting agents,
f. optionally adjusting the volume of "step d" or "step e" solution using non-aqueous solvents to get a clear bulk solution,
g. filtering the bulk solution through 0.22µ filter and
h. filling of filtered bulk solution in a vial.

In one another embodiment, the present invention provides process for preparing a stable liquid pharmaceutical composition comprising steps of:
a. providing a first quantity of water for injection in a glass beaker,
b. adding non-aqueous solvent to "step a" water,
c. adding micafungin Sodium to "step b" solution,
d. optionally adjusting pH of "step c" solution in the range of pH 4.0-7.0 using suitable pH adjusting agents,
e. optionally adjusting the volume of "step d" solution using second quantity of water for injection to get a clear bulk solution,
f. filtering the bulk solution through 0.22µ filter and
g. filling of filtered bulk solution in a vial.

The invention is further illustrated by the following examples which are provided to be exemplary of the invention and do not limit the scope of the invention. While the present invention has been described in terms of its specific embodiments, certain modifications and equivalents will be apparent to those skilled in the art and are intended to be included within the scope of the present invention.

### Abbreviations

CRT = Controlled room temperature

Impurity I, Impurity II, Impurity III, Impurity IV, Impurity V, Impurity VI and Impurity VII are described below.

### Impurity I

### Sodium 5-{(1S,2S,3S)-4-[(1S,2R)-[4-amino-1-[[(2S,3S,4S)-2-carbamoyl-3-hydroxy-4-methyl-1-pyrrolidinyl]carbonyl]-2-hydroxy-4-oxobutyl]amino]-3-[[[(2S,4R)-1-[(2S,3R)-2-[[[(4S)-4,5-dihydroxy-1-[4-[5-[4-(pentyloxy)phenyl] isoxazol-3-yl]benzoyl]-2-pyrrolidinyl]carbonyl]amino]-3-hydroxybutanoyl]-4-hydroxy-2-pyrrolidinyl]carbonyl]amino]-1,2-dihydroxy-4-oxobutyl}-2-hydroxy phenyl sulfate

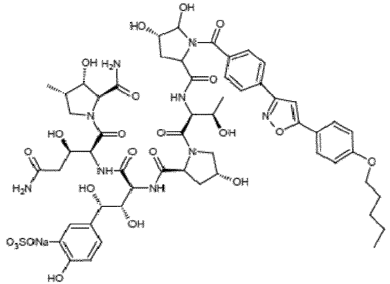

### Impurity II

### Sodium 5-[(1S,2S)-2-[(3S,6S,9S,11R,15S,18S,20R,21R,24S,25S)-3-[(R)-2-carbamoyl-1-hydroxyethyl]-11,20,21,25-tetrahydroxy-15-[(R)-1-hydroxyethyl]-2,5,8,14,17,23-hexaoxo-18-[4-[5-(4-pentyloxyphenyl)isoxazol-3-yl]benzoyl amino]-1,4,7,13,16,22-hexaazatricyclo[22.3.0.09,13]heptacos-6-yl]-1,2-dihydroxyethyl]-2-hydroxyphenyl sulfate

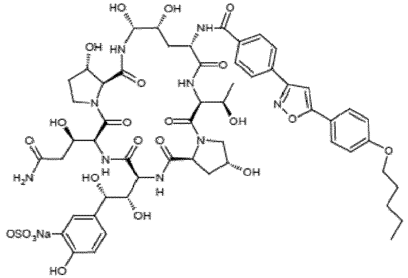

### Impurity III

### Sodium 5-[(1S,2S)-2-[(3S,6S,9S,11R,15S,18S,20R,21S,24S,25S,26S)-3-[(R)-2-carbamoyl-1-hydroxyethyl]-11,20,21,25-tetrahydroxy-15-[(R)-1-hydroxy ethyl]-26-methyl-2,5,8,14,17,23-hexaoxo-18-[4-[5-(4-pentyloxyphenyl) isoxazol-3-yl]benzoylamino]-1,4,7,13,16,22-hexaazatricyclo[22.3.0.09,13] heptacos-6-yl]-1,2-dihydroxyethyl]-2-hydroxyphenyl sulfate

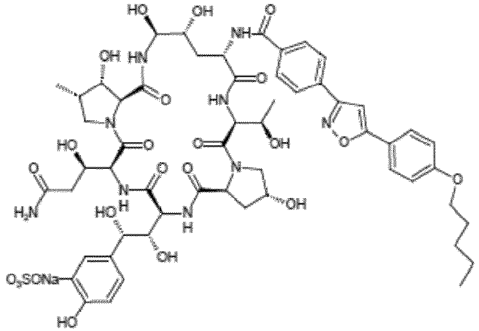

### Impurity IV

### N-{[(3S,6S,9S,11R,15S,18S,20R,21R,24S,25S,26S)-3-[(R)-2-carbamoyl-1-hydroxyethyl]-11,20,21,25-tetrahydroxy-15-[(R)-1-hydroxyethyl]-6-[(1S,2S)-1,2-dihydroxy-2-(3,4-dihydroxyphenyl)ethyl]-26-methyl-2,5,8,14,17,23-hexaoxo-1,4,7,13,16,22-hexaazatricyclo[22.3.0.09,13]heptacos-18-yl]}-4-[5-(4-pentyloxyphenyl)isoxazol-3-yl]benzamide

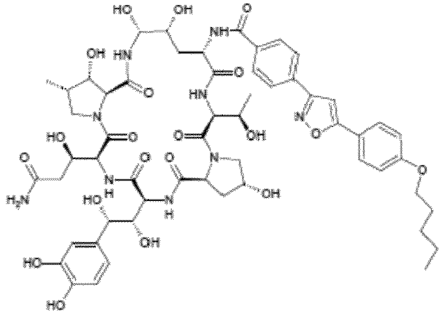

### Impurity V

### Sodium 5-[(1S,2S)-2-[(3S,6S,9S,11R,15S,18S,20R,21R,24S,25S,26S)-3-[(R)-2-carbamoyl-1-hydroxyethyl]-11,20,21,25-tetrahydroxy-15-[(R)-1-hydroxyethyl]-26-methyl-2,5,8,14,17,23-hexaoxo-18-[4-[3-(4-pentyloxyphenyl isoxazol-5-yl]benzoylamino]-1,4,7,13,16,22-hexaazatricyclo[22.3.0.09,13] heptacos-6-yl]-1,2-dihydroxyethyl]-2-hydroxyphenyl sulfate

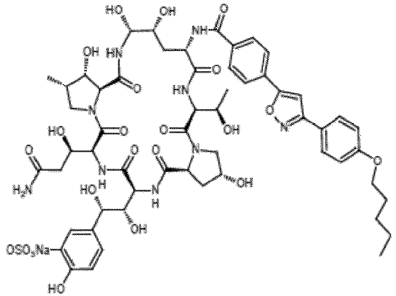

### Impurity VI

### Sodium 5-((1S,2S)-2-((3S,6S,7R,11R,12R,14S,17S,21R,22aS)-6-((2S,3S,4S)-2-carbamoyl-3-hydroxy-4-methylpyrrolidine-1-carbonyl)-7,11,12,21-tetrahydroxy-17-((R)-1-hydroxyethyl)-1,4,9,15,18-pentaoxo-14-(4-(5-(4-(pentyloxy)phenyl)isoxazol-3-yl)benzamido)docosahydropyrrolo[1,2-d][1,4,7,10,15]pentaazacycloicosin-3-yl)-1,2-dihydroxyethyl)-2-hydroxyphenyl sulfate

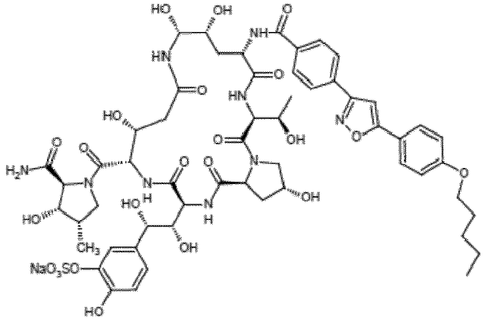

### Impurity VII

### Sodium 5-[(1S,2S)-2-[(3S,6S,9S,11R,15S,18S,21R,24S, 25S,26S)-3-[(R)-2-carbamoyl-1-hydroxyethyl]-11,21,25-trihydroxy-15-[(R)-1-hydroxyethyl]-26-methyl-2,5,8,14,17,23-hexaoxo-18-[4-[5-(4-pentyloxyphenyl)isoxazol-3-yl] benzoylamino]-1,4,7,13,16,22-hexaazatricyclo[22.3.0.09,13]heptacos-6-yl]-1,2-dihydroxyethyl]-2-hydroxyphenyl sulfate

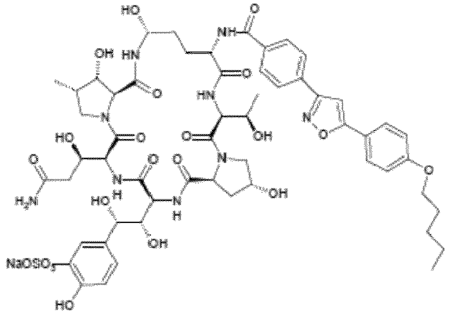

### Examples:

### Example 1:

| Sr. No. | Ingredient | Quantity |
|---|---|---|
| 1 | Micafungin Sodium | 100 mg/mL |
| 2 | N,N-Dimethylacetamide | Q.S. To 1 mL |

Process:
1. Approximately 80% quantity of N,N-Dimethylacetamide was taken in a glass beaker and stirred with nitrogen sparging.
2. Micafungin sodium was added under continuous stirring, and further stirred to dissolve.
3. Remaining quantity of N,N-Dimethylacetamide was added to make up the volume of the solution to batch size and mixed.
4. Bulk solution was prepared and filtered through 0.22µ filter.
5. The filtered bulk solution was filled in amber vial and the vial was closed . The vial was closed such that the headspace oxygen level in the vial was less than 10 %.

### Stability data

| Parameter | Example 1 | | | |
|---|---|---|---|---|
| | 2 - 8°C | | | |
| | Initial | 2M | 5M | 7M |
| Assay | 115.9 | 114.1 | 111.5 | 110.1 |
| Impurity I | 0.17 | 0.29 | 0.36 | 0.35 |
| Impurity at RRT 0.77 | ND | 0.05 | 0.06 | 0.06 |
| Impurity V | 0.05 | 0.04 | 0.04 | 0.03 |
| Impurity II | 0.07 | 0.1 | 0.08 | 0.08 |
| Impurity VII | 0.15 | 0.12 | 0.1 | 0.1 |
| Impurity VI | 0.07 | 0.08 | 0.06 | 0.06 |
| Impurity III | ND | ND | ND | ND |
| Impurity IV | ND | ND | ND | ND |
| Unknown impurity at 1.49 RRT | - | - | - | 0.05 |
| Total Impurities | 0.51 | 0.68 | 0.8 | 0.8 |

### Example 2:

| Sr. No. | Ingredient | Quantity |
|---|---|---|
| 1 | Micafungin sodium | 100 mg/mL |
| 2 | Polyethylene glycol 300 | 0.1 mL |
| 3 | N,N-Dimethylacetamide | Q.S. to 1 mL |

Process:
1. Approximately 50% quantity of N,N-Dimethylacetamide was taken in a glass beaker and stirred with nitrogen sparging.
2. Complete quantity of polyethylene glycol 300 was added under continuous stirring, and further stirred to dissolve.
3. Micafungin sodium was added under continuous stirring, and further stirred to dissolve.
4. Remaining quantity of N,N-Dimethylacetamide was added to make up the volume of the solution to batch size and mixed.
5. Bulk solution was prepared and filtered through 0.22µ filter.
6. The filtered bulk solution was filled in amber vial and the vial was closed. The vial was closed such that the headspace oxygen level in the vial was less than 10 %.

### Stability data

| Parameter | Example 2 (2 - 8°C) | | | |
|---|---|---|---|---|
| | Initial | 2M | 5M | 8M |
| Assay | 114.4 | 113.2 | 110.6 | 108 |
| Impurity I | 0.2 | 0.22 | 0.25 | 0.25 |
| Impurity at RRT 0.77 | 0.02 | 0.04 | 0.04 | 0.05 |
| Impurity V | 0.03 | 0.05 | 0.04 | 0.04 |
| Impurity II | 0.09 | 0.1 | 0.08 | 0.05 |
| Impurity VII | 0.15 | 0.12 | 0.1 | 0.09 |
| Impurity VI | 0.04 | 0.1 | 0.07 | 0 |
| Impurity III | ND | ND | 0.02 | 0.01 |
| Impurity IV | 0.12 | ND | 0.72 | 1.8 |
| Unknown impurity at 1.30 RRT | - | 0.52 | 0.02 | 0.1 |
| Total Impurities | 0.65 | 1.2 | 1.4 | 2.6 |

### Example 3:

| Sr. No. | Ingredient | Quantity (mg/mL) | % W/V |
|---|---|---|---|
| 1 | Micafungin sodium | 100 | 10 |
| 2 | Polyethylene glycol 300 | 500 | 50 |
| 3 | Propylene glycol | Q.S. to 1 mL | 40 |

Process:
1. Complete quantity of polyethylene glycol 300 was taken in a glass beaker and stirred with nitrogen sparging.
2. Micafungin sodium was added under continuous stirring, and further stirred to dissolve.
3. Complete quantity of propylene glycol was added to make up the volume of the solution to batch size and mixed.
4. Bulk solution was prepared and filtered through 0.22µ filter.
5. The filtered bulk solution was filled in an amber vial and the vial was closed. The vial was closed such that the headspace oxygen level in the vial was less than 10 %.

### Stability data

| Related substances | Micafungin sodium Injection 100 mg/mL, 1 mL: 2 M Stability Results | | |
|---|---|---|---|
| | Example 3 | | |
| | Initial | 2-8°C | |
| | | 1M | 2M |
| Impurity I | 0.06 | 0.21 | 0.26 |
| Impurity II + V | 0.09 | 0.12 | 0.1 |
| Impurity at RRT 1.04 | 0.27 | 0.31 | 0.38 |
| Impurity III | 0.01 | ND | 0.03 |
| Impurity IV | 0.08 | 0.57 | 1.4 |
| Impurity VI | ND | ND | ND |
| Impurity VII | ND | ND | ND |
| Impurity at RRT 0.77 | ND | ND | ND |
| Total impurities | 0.62 | 1.27 | 2.3 |

### Example 4:

| Sr. No. | Ingredient | Quantity |
|---|---|---|
| 1 | Micafungin sodium | 50 mg/mL |
| 2 | Propylene glycol | Q.S. to 1 mL |

Process:
1. Approximately 80% quantity of propylene glycol was taken in a glass beaker and stirred with nitrogen sparging.
2. Micafungin sodium was added under continuous stirring, and further stirred to dissolve.
3. Remaining quantity of propylene glycol was added to make up the volume of the solution to batch size and mixed.
4. Bulk solution was prepared and filtered through 0.22µ filter.
5. The filtered bulk solution was filled in an amber vial and the vial was closed. of the vial was closed such that the headspace oxygen level in the vial was less than 10 %.

### Stability data

| Parameter | | Example 4 | | | |
|---|---|---|---|---|---|
| | Initial | 2 - 8°C | | | |
| | | 1M | 3M | 6M | 12M |
| Assay | 100.8 | 101.2 | 100.1 | 98.8 | 94.4 |
| Impurity I | 0.22 | 0.15 | 0.16 | 0.16 | 0.15 |
| Impurity at RRT 0.77 | 0.04 | 0.02 | 0.02 | 0.03 | 0.03 |
| Impurity V | 0.04 | 0.05 | 0.04 | 0.03 | 0.03 |
| Impurity II | 0.08 | 0.07 | 0.08 | 0.06 | 0.06 |
| Impurity VII | 0.03 | 0.11 | 0.1 | 0.08 | 0.07 |
| Impurity VI | 0.05 | 0.06 | 0.06 | 0 | 0.04 |
| Impurity III | 0.02 | ND | 0.01 | 0.01 | ND |
| Impurity IV | 0.09 | 0.05 | 0.06 | 0.05 | 0.14 |
| Unknown impurity at 1.49 RRT | - | - | - | 0.05 | 0 |
| Total Impurities | 0.72 | 0.57 | 0.54 | 0.5 | 0.52 |

### Example 5:

### Multi dose vials

| Sr. No. | Ingredient | Quantity |
|---|---|---|
| 1 | Micafungin sodium eq. to micafungin | 50 mg/mL |
| 2 | Benzyl alcohol | 10 mg/mL |
| 2 | Propylene glycol | Q.S. to 1 mL |

Process:
1. Approximately 90% quantity of propylene glycol was taken in a glass beaker and stirred with nitrogen sparging.
2. Complete quantity of benzyl alcohol was added and stirred to dissolve.
3. Micafungin sodium was added under continuous stirring, and further stirred to dissolve.
4. Remaining quantity of propylene glycol was added to make up the volume of the solution to batch size and mixed.
5. Bulk solution was prepared and filtered through 0.22µ filter.
6. The filtered bulk solution was filled in amber vial and the vial was closed. The vial was closed such that the headspace oxygen level in the vial was less than 10 %.

### Stability data

| Parameter | | Example 5 | |
|---|---|---|---|
| | Initial | 2 - 8°C | |
| | | 1M | 3M |
| Assay | 109.8 | 109.9 | 106.5 |
| Impurity I | 0.18 | 0.59 | 0.13 |
| Impurity at RRT 0.77 | 0.03 | 0.1 | 0.03 |
| Impurity V | 0.04 | 0.04 | 0.04 |
| Impurity II | 0.08 | 0.08 | 0.07 |
| Impurity VII | 0.1 | 0.14 | 0 |
| Impurity VI | 0.03 | 0.02 | 0.1 |
| Impurity III | 0 | 0 | 0.02 |
| Impurity IV | 0 | 0 | 0 |
| Unknown impurity at 1.15 RRT | - | 0.19 | |
| Unknown impurity at 1.30 RRT | - | - | 0.06 |
| Unknown impurity at 1.49 RRT | 0.02 | - | |
| Total Impurities | 0.53 | 1.3 | 0.57 |

### Example 6:

| Sr No | Ingredient | Quantity |
|---|---|---|
| 1 | Micafungin sodium | 10 mg/mL |
| 2 | Propylene glycol | 0.5 mL |
| 3 | Citric acid | q.s. to pH 5.5 |
| 4 | Sodium hydroxide | q.s. to pH 5.5 |
| 5 | Water for injection | q.s. to 1 mL |

Process:
1. Approximately 40% quantity of water for injection was taken in a glass beaker and stirred with nitrogen sparging.
2. Complete quantity of propylene glycol was added under continuous stirring, and further stirred to dissolve.
3. Micafungin sodium was added under continuous stirring, and further stirred to dissolve.
4. pH of the resultant solution was adjusted to 5.5 using sodium hydroxide and/or citric acid solution.
5. Remaining quantity of water for injection was added to make up the volume of the solution to batch size and mixed.
6. Bulk solution was filtered through 0.22µ filter.
7. The filtered bulk solution was filled in amber vial and the vial was closed such that the headspace oxygen level in the vial was less than 10 %.

### Stability data

| Parameter | Example 6 | | | |
|---|---|---|---|---|
| | Initial | 2 - 8°C | | |
| | | 1M | 2M | 6M |
| Assay | 114.7 | 114.8 | 110.8 | 109.1 |
| Impurity I | 0.13 | 0.16 | 0.27 | 0.19 |
| Impurity at RRT 0.77 | 0.02 | 0.02 | 0 | 0.03 |
| Impurity V | 0.05 | 0.04 | 0 | 0.04 |
| Impurity II | 0.09 | 0.06 | 0.09 | 0.06 |
| Impurity VII | 0.12 | 0.1 | 0.13 | 0.1 |
| Impurity VI | 0.07 | 0 | 0.1 | 0.01 |
| Impurity III | 0.02 | 0.02 | 0 | 0.01 |
| Impurity IV | 0.02 | ND | 0 | 0.03 |
| Unknown impurity | ND | ND | ND | ND |
| Total Impurities | 0.58 | 0.53 | 0.59 | 0.39 |

While the invention has been described in terms of its specific embodiments, certain modifications and equivalents will be apparent to those skilled in the art and are intended to be included within the scope of the invention.

## Claims

1. A liquid pharmaceutical composition for parenteral administration comprising micafungin, one or more non-aqueous solvents, and optionally water.

2. The liquid pharmaceutical composition according to claim 1, wherein the non-aqueous solvent is a polar aprotic solvent.

3. The liquid pharmaceutical composition according to claim 2, wherein the polar aprotic solvent comprises one or more of dimethylacetamide, 1-methyl-2-pyrrolidone, 1,3-dimethyl-2-imidazolidinone, dimethyl sulfoxide, acetone, tetrahydrofuran, 1,4-dioxane, acetonitrile, dimethyl formamide, and propylene carbonate.

4. The liquid pharmaceutical composition according to claim 1, wherein the non-aqueous solvent is a polar protic solvent.

5. The liquid pharmaceutical composition according to claim 4, wherein the polar protic solvent comprises one or both of propylene glycol, and polyethylene glycol.

6. The liquid pharmaceutical composition according to claim 1, wherein the micafungin is present in the form of micafungin sodium.

7. The liquid pharmaceutical composition according to claim 6, wherein the micafungin sodium is present in a concentration from about 10 mg/mL to about 200 mg/mL.

8. The liquid pharmaceutical composition according to claim 1, wherein the composition retains at least 80% of the potency of micafungin after storage for three months at 2-8°C.

9. The liquid pharmaceutical composition according to claim 1, wherein the composition does not contain total impurities more than 3% as determined by high performance liquid chromatography after storage for three months at 2-8°C.

10. The liquid pharmaceutical composition according to claim 1 further comprising citric acid and/or sodium hydroxide, wherein the composition has a pH between about 4 and about 7.
